# FASCICULE DE BREVET EUROPEEN

(11) **EP 1 368 478 B1**
(45) Date de publication et mention de la délivrance du brevet: **26.04.2006**
(21) Numéro de dépôt: 02710945.3
(22) Date de dépôt: 04.01.2002
(51) Int. Cl.: C12N 15/49

(54) **POLYPEPTIDE INDUISANT DES ANTICORPS NEUTRALISANT LE VIH**
POLYPEPTID, DAS NEUTRALISIERENDE ANTIKÖRPER GEGEN HIV INDUZIERT
POLYPEPTIDE INDUCING HIV-NEUTRALISING ANTIBODIES

(30) Priorité: 05.01.2001 FR 0100141; 23.01.2001 FR 0100848
(43) Date de publication de la demande: 10.12.2003
(73) Titulaire: Sanofi Pasteur, 69367 Lyon Cedex 07 (FR)
(72) Inventeur: BRASSEUR, Robert, B-5351 Haillot (BE); CHARLOTEAUX, Benoît, B-5000 Namur (BE); CHEVALIER, Michel, F-38270 Beaurepaire (FR); EL HABIB, Raphaelle, F-69630 Chaponost (FR); KRELL, Tino, F-69130 Ecully (FR); SODOYER, Régis, F-69110 Sainte-Foy-les-Lyon (FR)
(74) Mandataire: Schaeffer, Nathalie Christiane
(86) Numéro de dépôt international: PCT/FR2002/000031
(87) Numéro de publication internationale: WO 2002/053587

(56) Documents cités:
- WO-A-00/08043
- WO-A-00/40616
- WENG Y. ET AL.: "Mutational analysis of residues in the coiled-coil domain of human immunodeficiency virus type 1 transmembrane protein gp41." JOURNAL OF VIROLOGY, vol. 72, no. 12, décembre 1998 (1998-12), pages 9676-9682, XP002180284 ISSN: 0022-538X
- CAO J. ET AL.: "Effects of amino acid changes in the extracellular domain of the human immunodeficiency virus type 1 gp41 envelope glycoprotein." JOURNAL OF VIROLOGY, vol. 67, no. 5, 1993, pages 2747-2755, XP001030658 ISSN: 0022-538X
- WENG Y. ET AL.: "Structure-function studies of the self-assembly domain of the human immunodeficiency virus type 1 transmembrane protein gp41." JOURNAL OF VIROLOGY, vol. 74, no. 11, juin 2000 (2000-06), pages 5368-5372, XP002218221 ISSN: 0022-538X
- LACASSE R. ET AL.: "Fusion-competent vaccines: Broad neutralization of primary isolates of HIV" SCIENCE, vol. 283, no. 5400, 15 janvier 1999 (1999-01-15), pages 357-362, XP002120812 ISSN: 0036-8075
- WEISSENHORN W. ET AL.: "ATOMIC STRUCTURE OF THE ECTODOMAIN FROM HIV-1 GP41" NATURE, vol. 387, no. 6631, 22 mai 1997 (1997-05-22), pages 426-430, XP001002849 ISSN: 0028-0836 cité dans la demande
- MALASHKEVICH V. N. ET AL.: "Crystal structure of the simian immunodeficiency virus (SIV) gp41 core: Conserved helical interactions underlie the broad inhibitory activity of gp41 peptides" PROCEEDINGS OF THE NATIONAL ACADEMY OF SCIENCES OF USA, NATIONAL ACADEMY OF SCIENCE. WASHINGTON, US, vol. 95, no. 16, 4 août 1998 (1998-08-04), pages 9134-9139, XP002170630 ISSN: 0027-8424
- MONTEFIORI D. ET AL.: "Toward an HIV type 1 vaccine that generates potent, broadly cross-reactive neutralizing antibodies" AIDS RESEARCH AND HUMAN RETROVIRUSES, vol. 15, no. 8, 20 mai 1999 (1999-05-20), pages 689-698, XP002144409 ISSN: 0889-2229

## Description

La présente invention se rapporte à un polypeptide muté dérivant de la protéine gp41, ainsi qu'à un vaccin le comprenant.

La mise au point d'une méthode d'immunisation contre le VIH est aujourd'hui l'une des priorités de la recherche scientifique.

Les obstacles majeurs que sont la grande variabilité génétique du virus et la faible exposition au système immunitaire d'épitopes viraux neutralisants freinent considérablement l'élaboration d'une immunité neutralisante.

La glycoprotéine d'enveloppe du VIH qui est requise pour conférer au virus son caractère infectieux représente la cible d'anticorps neutralisants. Ces caractéristiques ont fait de cette dernière un sujet d'investigations intenses.

L'enveloppe glycoprotéique (env) du virus d'immunodéficience humaine-1 (VIH-1) est synthétisée à partir du précurseur gp160 qui donne sous l'action d'une protéase les sous-unités gp120 et gp41.

La fixation de gp120/gp41 aux récepteurs cellulaires (CD4 et un récepteur des chimiokines tel que CCR5 ou CXCR-4) induit un changement de conformation de la gp41 d'un état latent (non-fusogène) à un état fusion-actif (fusogène). Entre ces deux états, existe un état transitoire dit "intermédiaire" pendant lequel la gp41 se présente comme une protéine membranaire à la fois dans les membranes virale et cellulaire (Weissenhorn et al Nature (1997), 387 (6631), 426-30).

Des expériences de liaison ont permis d'établir que l'état latent non-fusogène est caractérisé par l'inaccessibilité de larges portions de l'ectodomaine de la gp41. La gp120 interagit en effet de sorte à masquer les épitopes. Il a par ailleurs été montré que l'inhibition du changement de structure de l'état intermédiaire vers l'état fusogène par des peptides utilisés en tant que compétiteurs pouvait affecter l'infection virale, (Weissenhom W. et al, Molecular Membrane Biology, 1999, 16, 3-9).

L'utilisation de l'état fusogène de la gp41 à des fins vaccinales est décrite dans WO00/40616. Selon cette demande, les hélices N peuvent être utilisées seules ou en association avec les hélices C pour reproduire dans ce dernier cas la conformation fusogène de la gp41.

D'autre part, l'effet de l'introduction de certaines mutations dans le domaine proche du peptide de fusion de la gp41 (aa.553-595) a été décrit par Weng, Y. et al. (J. of Virology, vol.72, No. 12, pages 9676-9682, Dec.1998).

La demanderesse propose un nouvel antigène vaccinal utilisable dans la vaccination contre le VIH. La demanderesse a en effet mis en évidence pour la première fois que l'état intermédiaire de la gp41 est capable d'induire des anticorps neutralisant les isolats primaires du VIH.

La présente invention concerne donc un polypeptide capable de former une structure correspondant à ou mimant l'état intermédiaire de la gp41.

Selon un mode de réalisation, ledit polypeptide comprend au moins une mutation sélectionnée dans le groupe consistant en : I101D ou S.

Selon un mode de réalisation particulier, le polypeptide comprend au moins une autre mutation sélectionnée dans le groupe comprenant les mutations suivantes: G13A, L, M, I, W ou K ; Q17A ou E ; Q18A ou E; A24Q, E, S ou R ; Q28A ; T35I ou L ; V36Q ou E ; W37S ou D ; G38A, V, L, I, M ou E ; Q39A, V, I, I, M ou E ; K40E, A, V, L, I ou M ; Q41A, V, L, I, M ou E ; Q43A, V, L, I, M ou E ; L47A ou D ; V49I ou L ; R51A, N ou E ; Q56I ; C64S ; C70S ; ou L ; W94D ; D98A, V, L, I, M ou K ; R99A, N ou E ; Y104M ou E ; I108D ; Q119A, V, L, I, M, S, N ou R ; E120A ; K121A ; E123A ; E125A ; R153N ou A et R173N ou A.

Selon un mode de réalisation préféré, le polypeptide selon la présente invention comprend les mutations suivantes : T35I + Q28I +I101D ; T35I + Q28I + I101D + Q119N ; I101D + I108D + Q131N + W37A ou I101D + I108D + Q142N + L126D ; W37A + I101D + I108D + Q119N ; ou I101D + I108D + Q119N + L126D.

Selon un autre aspect la présente invention concerne un conjugué comprenant un polypeptide selon l'invention conjugué à une protéine ou un peptide porteur.

Selon un autre aspect, la présente invention concerne une séquence d'ADN codant pour un polypeptide selon l'invention ou pour un conjugué selon l'invention.

La présente invention concerne également un vecteur d'expression comprenant ladite séquence d'ADN ainsi qu'une cellule-hôte contenant ledit vecteur.

La présente invention a également pour objet un vaccin contre le VIH, comprenant au moins un polypeptide tel que défini ci-dessus, ou au moins un conjugué tel que défini ci-dessus, ou au moins un vecteur d'expression tel que défini ci-dessus, un support pharmaceutiquement acceptable et éventuellement un adjuvant.

Un autre objet de la présente invention a trait au procédé de préparation d'un polypeptide tel que défini ci-dessus comprenant l'expression dudit polypeptide à partir d'une cellule-hôte telle que définie ci-dessus.

L'invention est décrite plus en détails dans la description qui suit.

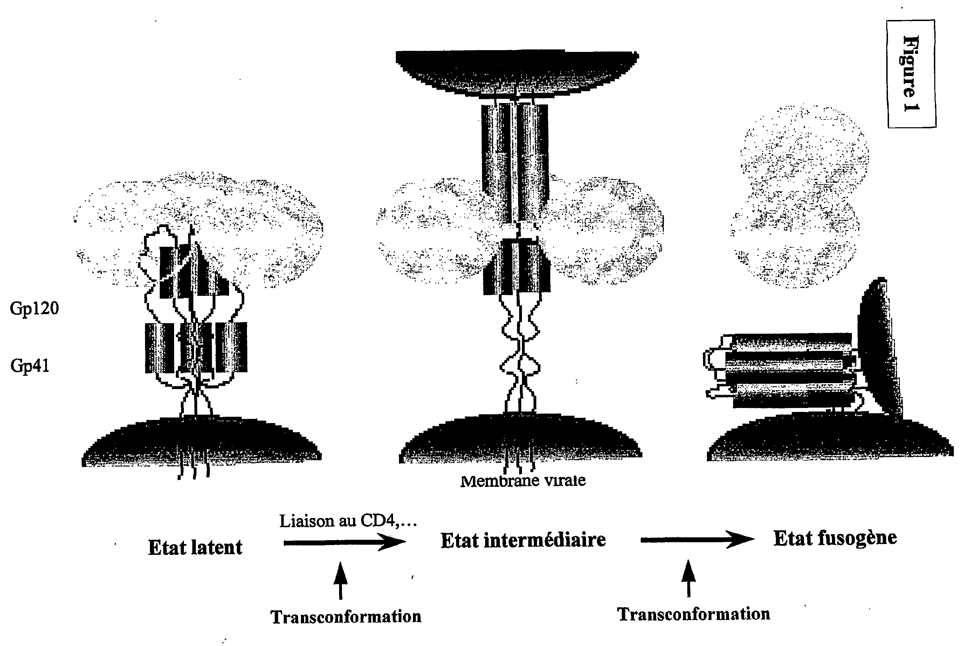

Le phénomène de changement de conformation de la gp41 précédant la fusion des membranes cellulaires et virales est illustré à la figure 1.

La demanderesse a mis évidence de façon surprenante que l'état intermédiaire de la gp41 induisait chez l'homme des anticorps neutralisant les isolats primaires du VIH. L'induction d'anticorps neutralisant les isolats primaires peut être aisément déterminée par le test de neutralisation tel que décrit dans l'article de C. Moog et al (AIDS Research and human retroviruses, vol. 13(1), 13-27, 1997). On estime dans le cadre de la présente invention que des anticorps neutralisants ont été induits par l'antigène testé lorsque le sérum dilué au 1/5^{ème} entraîne une réduction d'un facteur 10 de la quantité de p24 présente dans le surnageant de culture.

On entend dans le cadre de la présente invention par « polypeptide correspondant ou mimant l'état intermédiaire de la gp41 » un polypeptide, de préférence trimérique, présentant dans des conditions physiologiques une conformation ouverte. Cette conformation ouverte se caractérise par le fait qu'au moins l'une des hélices C n'est pas appariée autour des hélices N selon l'orientation anti-parallèle telle que présente dans la forme fusogène. De préférence, les trois hélices C ne sont pas appariées aux hélices N selon l'orientation anti-parallèle telle que présente dans la forme fusogène. Dans une telle conformation, il est probable que l'hélice C non appariée au trimère central constitué des hélices N adopte une conformation libre (« coil »). Dans la conformation ouverte selon l'invention, les hélices N sont appariées entre elles selon une orientation parallèle en formant de préférence un trimère. Dans le cas du monomère, la conformation ouverte selon l'invention se caractérise par le fait que l'hélice C n'est pas appariée à l'hélice N.

L'obtention d'une conformation ouverte peut être mise en évidence par la technique de mesure de la fluorescence intrinsèque du polypeptide telle que décrite par Schmid, F.X. (1989) « Spectral methods of characterising protein conformation and conformational changes » Creighton, T.E. Protein structure- a practical approach. pp251-284, IRL,Oxford University Press. En résumé, le polypeptide va être excité à 295 nm et le spectre d'émission de la fluorescence va être enregistrée à 310-380 nm. Il y a une corrélation entre le maximun d'émission à ces longueurs d'onde et l'environnement des tryptophanes dans la structure. Un résidu tryptophane qui est complètement exposé au solvant (i.e. environnement hydrophile) a un maximum d'émission d'environ 355 nm, alors qu'un résidu tryptophane qui est protégé du solvant (*i.e.* présent à l'intérieur du polypeptide) a un maximum d'émission d'environ 325 nm. Le polypeptide selon l'invention présente dans sa forme trimérique 9 résidus tryptophane au niveau des interfaces N/C. L'obtention d'une conformation ouverte va donc se traduire par une augmentation du maximum d'émission enregistré à 310-380 nm.

Le polypeptide selon l'invention a la particularité d'être stable c'est à dire qu'il conserve sa conformation intermédiaire en conditions physiologiques. La stabilité du peptide selon l'invention peut être aisément contrôlée par la technique de microcalorimétrie différentielle (DSC) bien connue de l'homme de l'art. On peut se référer par exemples aux articles de A. Cooper et al, Phil Trans. R. Soc. Lon. A (1993) 345, 23-35, et de V.V.Plotnikov et al Analytical Biochemistry 250, 237-244, (1997).

La demanderesse a également mis en évidence de façon surprenante que le polypeptide selon l'invention conservait sa conformation « ouverte » en milieu fortement acide. Cette propriété fait du polypeptide selon l'invention un antigène vaccinal administrable par voie orale. La demanderesse a en effet montré que l'ectodomaine de la protéine gp41 est extrêmement thermostable à pH2,5. La mesure du Tm (température à laquelle 50% des protéines présentes sont dénaturées) de la gp41 dans 50mM de formate de sodium par DSC (calorimétrie différentielle à balayage) donne une valeur de 110°C, le début du phénomène de dénaturation apparaissant à environ 100°C à pH=2,5. La thermostabilité de la protéine gp41 à pH neutre a été évaluée par Weissenhorn et al (EMBO, 1996, 7, 1507-1514). Le Tm mesuré à pH neutre par ces auteurs est de 78°C, ce qui signifie que de façon surprenante cette protéine est plus stable à pH acide qu'à pH neutre. Ces résultats ont été confirmé par une analyse par dichroïsme circulaire visant à calculer le pourcentage d'hélice alpha dans la protéine.
La demanderesse a également montré que le polypeptide selon l'invention présentait la même particularité. Cette propriété spécifique fait du polypeptide selon l'invention un antigène vaccinal de choix pour une administration par voie orale.

Le polypeptide selon l'invention est constitué de la séquence correspondant à la protéine gp41 dépourvue de tout ou partie, de préférence de la totalité, de la séquence correspondant au domaine transmembranaire. Le polypeptide selon la présente invention est en outre dépourvu de tout ou partie, de préférence de l'intégralité, de la séquence correspondant au peptide de fusion. Selon un mode de réalisation préféré, tout ou partie de la séquence correspondant au domaine intracytoplasmique est également délété.

On entend par « gp41 » dans le cadre de la présente invention, une protéine gp41 issue de toute souche du VIH1 ou du VIH2, de préférence du VIH1, incluant les souches de laboratoire et les isolats primaires. On peut citer à titre illustratif les souches MN et BX08.

La séquence nucléotique et peptidique d'un grand nombre de protéines gp41 est connue et disponible par exemple sur internet (http://hiv web.lanl.gov/) et dans les compendium de Los Alamos correspondants. II est clair que toute séquence dans laquelle une ou plusieurs mutations conservatives a été introduite est également incluse dans le cadre de la présente invention.

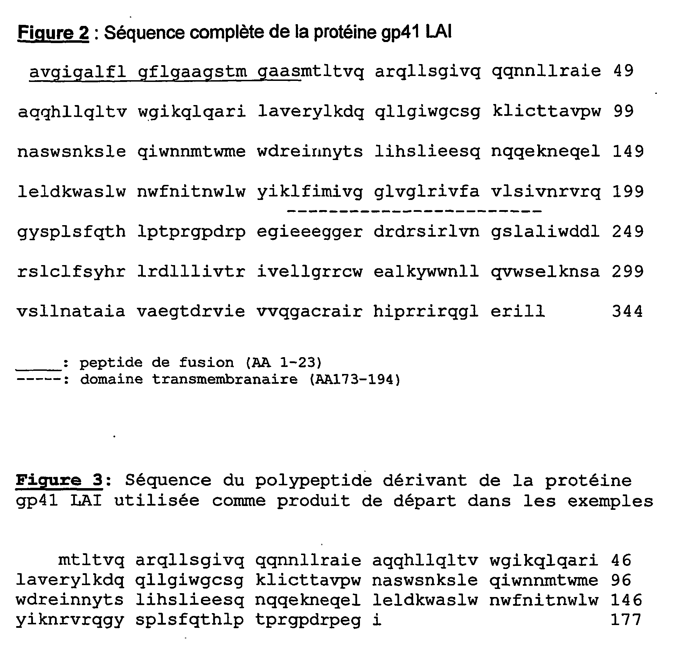

Les différents domaines constitutifs de la gp41 identifiés ci-dessus sont définis ici par référence à la séquence de la gp41 LAI telle que représentée à la figure 2 dans laquelle le 1^{er} acide aminé A est numéroté 1. Tous les auteurs ne s'accordent pas sur la définition des séquences correspondant au peptide de fusion et au domaine transmembranaire. Selon certains auteurs, le peptide de fusion correspond à la séquence 1-32.
Bien que la suppression de la séquence 1-23 soit préférée, en particulier du fait de la présence d'une méthionine en position 24, la suppression de la séquence 1-32 est également appropriée dans le cadre de la présente invention.
Concernant le domaine transmembranaire, certains auteurs estiment que ce dernier commence au résidu 154. Bien que la suppression de la séquence 173-194 soit préférée, la suppression de la séquence 154-194 est également envisagée dans le cadre de la présente invention.

Le polypeptide selon l'invention peut être obtenu par mutation de la séquence naturelle de la gp41.

Selon un mode de réalisation préféré, le polypeptide selon l'invention est préparé à partir de la séquence de la gp41 LAI dans laquelle le domaine transmembranaire et le peptide de fusion ainsi qu'une partie du domaine intracytoplasmique ont été supprimés. Cette séquence est représentée à la figure 3.

Les mutations identifiées dans la suite sont numérotés par référence à la séquence de la figure 3 dans laquelle le 1^{er} acide aminé M est numéroté 1.

La demanderesse a mis en évidence un certain nombre de mutations qui déstabilisent la structure de l'état latent et/ou de l'état fusogène de gp41 (à l'état monomérique et/ou trimérique) et/ou stabilisent l'état intermédiaire de gp41 (à l'état monomérique et/ou trimérique), et/ou favorisent la transconformation de l'état latent à l'état intermédiaire et/ou défavorisent la transconformation de l'état intermédiaire à l'état fusogène. Ces mutations permettent de stabiliser le polypeptide selon l'invention dans une conformation ouverte.

La demanderesse a mis en évidence que le polypeptide selon la présente invention et plus particulièrement les hélices N et C identifiées dans la structure à l'état fusogène pouvaient être divisées en quatre régions (numérotées par référence à la séquence de la séquence de la figure 3) correspondant respectivement aux séquences Ala7-Ile25 (région 1), Glu26-Ala44 (région 2), Arg45-Leu58 (région 3) et Trp94-Lys131 (région 4) et que les mutations modifiant les propriétés électrostatiques et/ou l'amphiphilicité et/ou les propriétés structurales de ces régions conduisent à l'obtention d'un polypeptide en conformation ouverte selon l'invention. La demanderesse a en particulier montré que la modification de la structure de la région 2, passant d'une structure étendue à une structure en hélice alpha, entraîne la formation de l'hélice N et l'obtention d'une conformation ouverte.
Les modifications introduites peuvent être évaluées par des méthodes classiques bien connues de l'homme de l'art. On peut citer par exemple à titre de méthode utilisable la méthode d'Eisenberg permettant la mesure de l'hydrophobicité moyenne et du moment hydrophobe moyen le long d'une séquence, l'analyse des clusters hydrophobes, des potentiels hydrophobes ou des potentiels électrostatiques moléculaires. Les modifications de structure peuvent être évaluées par exemple par des études de dynamique moléculaire ainsi que par des logiciels de prédictions de structures secondaires tels que PHD, NPSA, etc. Ces méthodes sont décrites dans les articles suivants : **Méthode d'Eisenberg :** Eisenberg, D., Schwarz, E., Komaromy, M and Wall, R 1984 *Journal of Molecular Biology.* 179 : 123-142 ; **Analyse des clusters hydrophobes :** Gaboriaud, C., Bissery, V., Benchetrit, T. and Mornon, JP. 1987. *FEBS Letters*. 224 (1) : 149-55; **Potentiel d'Hydrophobicité Moléculaire:** Brasseur, R 1991. *Journal of biological chemistry*. 266 : 16120-16127 ; **Potentiel Electrostatique Moléculaire :** Delleers, M. and Brasseur, R 1989 *Biochemical Pharmacology.* 38 (15) : 2441-2447 ; **Dynamique Moléculaire (Etude de la stabilité) :** Berendsen, H.J.C., van der Spoel, D. and van Drunen, R. 1995 GROMACS . *Computer Physics Communications.* 95 : 43-56 ; **Dynamique Moléculaire (Transconformation) :** Guilbert, C., Perahia, D. and Mouawad, L. 1995. *Computer Physics Communications.* 91 : 263-273 ; **PHD :** Rost, B. and Sander, C. 1994 *Proteins*. 19 : 55-72 ; **NPSA :** Combet, C., Blanchet, C., Geourjon, C. and Deléage, G. 2000 *Trends in biochemical sciences*. 25 (3) : 147-150.

La demanderesse a montré que les mutations telles que définies ci-dessus et portant sur des acides aminés de la région 1 défavorisent les interactions de cette région avec les régions 3 et 4 dans l'état latent et/ou avec la région 4 dans l'état fusogène et /ou favorisent la multimérisation de cette région dans l'état fusogène. Les mutations telles que définies ci-dessus et portant sur des acides aminés de la région 2 favorisent une structure de cette région en hélice α et/ou la transconformation en une telle structure, et/ou défavorisent les interactions de cette région avec les régions 3 et 4 dans l'état latent et/ou avec la région 4 dans l'état fiisogène et/ou favorisent la multimérisation de cette région dans l'état fusogène. Les mutations telles que définies ci-dessus et portant sur des acides aminés de la région 3 favorisent la multimérisation dans l'état latent et/ou dans l'état fusogène, et/ou défavorisent les interactions de cette région avec les régions 1 et 2 dans l'état latent et/ou avec la région 4 dans l'état fusogène. Les mutations telles que définies ci-dessus et portant sur des acides aminés de la région 4 favorisent la multimérisation de cette région dans l'état latent et/ou défavorisent les interactions de cette région avec les régions 1 et 2 dans l'état latent et/ou avec les régions 1, 2 et 3 dans l'état fusogène.

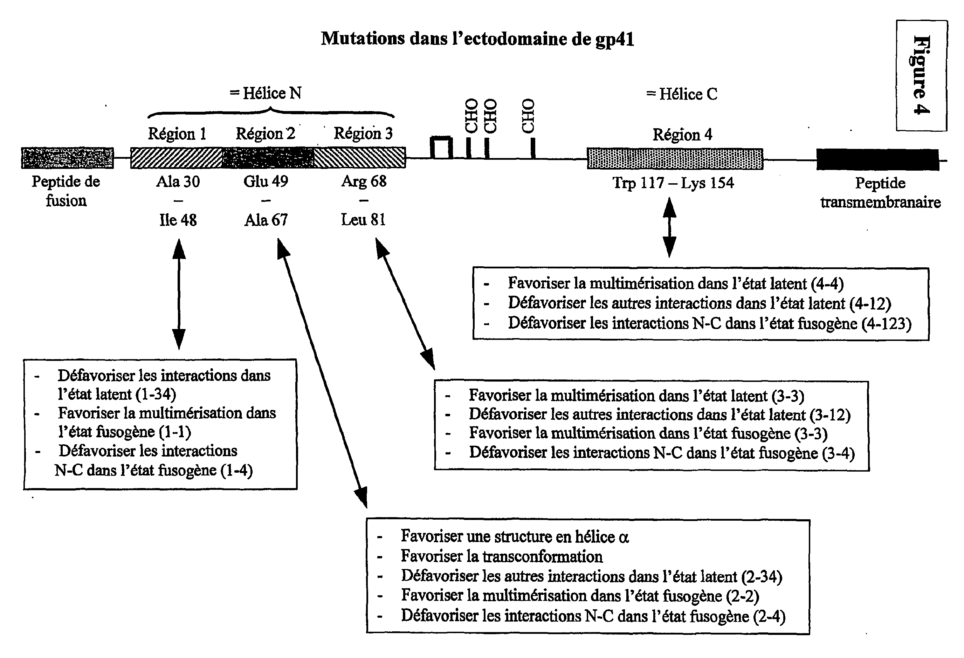

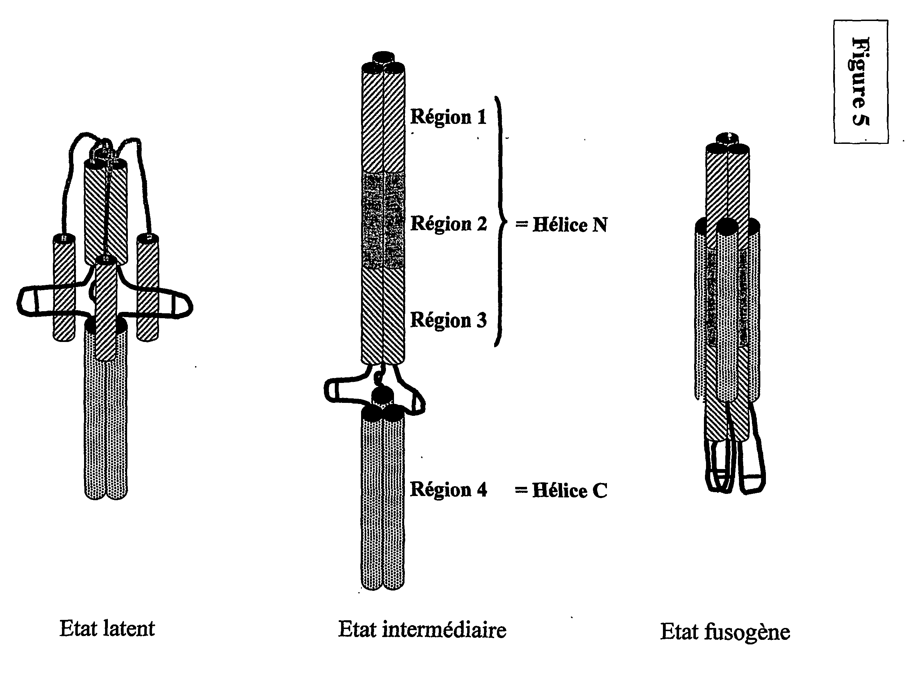

Les régions 1 à 4 du polypeptide selon l'invention ainsi que la fonction des mutations sont résumées dans les figures 4 et 5. Dans la figure 4, les régions 1 à 4 sont localisées dans la protéine gp41 complète incluant donc le peptide de fusion (AA 1-23) avec le 1^{er} acide aminé A numéroté 1).

Dans ce qui suit, les acides aminés sont représentés par leur code international, et le code L₁NNL₂ indique que l'acide aminé représenté par L₁ se trouvant en position NN est muté en l'acide aminé représenté par L₂.

La présente invention a donc pour objet un polypeptide qui comprend au moins une mutation, sélectionnée(s) dans le groupe consistant en: I101D ou S, de préférence deux mutations sélectionnée(s) dans le groupe consistant en :T35I et I101D ou S. Cette première mutation est de préférence associée à au moins une autre mutation, de préférence à 1 à 3 mutations, localisée(s) de préférence dans une ou plusieurs des régions 1 à 4. Dans le cas où deux mutations sont sélectionnées dans le groupe ci-dessus, ces mutations sont combinées à au moins une autre mutation, de préférence à 1 à 2 mutations, localisée(s) de préférence dans une ou plusieurs des régions 1 à 4.

Le polypeptide selon la présente invention comprend de préférence au moins deux mutations qui défavorisent les interactions entre les hélices N/C. Selon un aspect particulièrement préféré ces deux mutations sont combinées avec au moins une mutation, de préférence deux mutations, qui favorisent les interactions entre les hélices N selon une orientation parallèle. Ledit polypeptide ainsi obtenu peut avantageusement comporter en outre une ou plusieurs des autres mutations présentant les effets identifiés à la figure 4, et en particulier au moins une des mutations identifiées dans le tableau 1 aux colonnes 3 à 6.

Ces mutations supplémentaires sont de préférence sélectionnées dans le groupe comprenant les mutations suivantes : G13A, L, M, I, W ou K ; Q17A ou E ; Q18A ou E; A24Q, E, S ou R ; Q28A; T35I ou L ; V36Q ou E ; W37S ou D ; G38A, V, L, I, M ou E ; Q39A, V, L, I, M ou E ; K40E, A, V, L, I ou M ; Q41A, V, L, I, M ou E ; Q43A, V, L, I, M ou E ; L47A ou D ; V49I ou L ; R51A, N ou E ; Q56I; C64S ; C70S ; ou L ; W94D ; D98A, V, L, I, M ou K; R99A, N ou E ; Y104M ou E ; I108D ; Q119A, V, L, I, M, S, N ou R ; E120A ; K121A ; E123A ; E125A ; R153N ou A et R173N ou A.

De préférence les positions K40 et D98 ne sont pas mutées simultanément.

A titre d'exemple de combinaisons de mutations supplémentaires utilisables dans le cadre de la présente invention, on peut citer : Q17A + Q 18A ; Q17A + Q 18A + Q28A; Q41E + Q43E ; C64S + C70S ; E120A, + K121A ; E120A + E123A et K121A + E125A.

Des exemples de polypeptides préférés selon la présente invention sont les polypeptides comprenant les mutations suivantes: T35I + Q28I +I101D ; T35I + Q28I + I101D + Q119N ; ; V49I + Q28I +I101D ; V49I + Q28I + I101D + Q119N; Q56I + Q28I +I101D ; Q56I + Q28I + I101D + Q119N ; I101D ; I101S; I108D; W94D ; I101D + Q28I + V49I ; I101D + Q28I + Q56I ; I101S + Q28I + T35I ; I101S + Q28I + V49I ; I101S + Q28I + Q56I ; I101D + I108D + Q131N + W37A ; I101S+ I108D + Q131N + W37A; I101D + W94D + Q131N + W37A; I101S+ W94D + Q131N + W37A ; I101D + I108D + Q142N + L126D ; I101S+ I108D + Q142N + L126D ; I101D + W94D + Q142N + L126D ; I101S+ W94D + Q142N + L126D ; T35I + Q28I + I101S + Q119N; V49I + Q28I + I101S + Q119N ; Q56I + Q28I + I101S + Q119N ; T35L + Q28I +I101D ; T35L + Q28I + I101D + Q119N ; V49L + Q28I +I101D ; V49L + Q28I + I101D + Q119N ; Q56L + Q28I +I101D ; Q56L + Q28I + I101D + Q119N; I101D + Q28I + V49L ; I101D + Q28I + Q56L ; I101S + Q28I + T35L ; I101S + Q28I + V49L ; I101S + Q28I + Q56L ; T35L + Q28I + I101S + 119N; V49L + Q28I + I101S + Q119N ; Q56L + Q28I + I101S + Q119N; W37A + I101D + I108D + Q119N ; ou I101D + I108D + Q119N + L126D.

Ces polypeptides correspondent de préférence à un polypeptide de séquence SEQ ID N°2 dans lequel les mutations ci-dessus ont été introduites.

Des exemples de polypeptides particulièrement préférés dans le cadre de la présente invention sont les polypeptides de séquence SEQ ID N°2 dans lesquels les mutations: I101D ; T35I + Q28I +I101D ; T35I + Q28I + I101D + Q119N ; I101D + I108D + Q131N + W37A ou I101D + I108D + Q142N + L126D ; W37A + I101D + I108D + Q119N ; ou I101D + I108D + Q119N + L126D ont été introduites.

D'autres modifications peuvent être apportées au polypeptide de l'invention, par exemple pour faciliter l'expression et favoriser une meilleure solubilité. On peut avantageusement remplacer une ou les deux cystéines en position 64 et 69 par exemple par de'la sérine.

Bien que le polypeptide selon la présente invention présente une conformation ouverte qui soit stable en conditions physiologiques, cette conformation peut être renforcée par addition de résidus cystéines aux extrémités du polypeptide. A cette fin, deux résidus cystéine supplémentaires peuvent être ajoutés en N-terminal ou en C-terminal, de préférence en N-terminal du polypeptide selon l'invention pour fixer par covalence le trimère dans une conformation ouverte. Dans ce cas, les acides aminés Q17 et Q18 sont de préférence mutés en cystéine.

Les mutations proposées peuvent être combinées pour obtenir un effet de synergie ou tout au moins un effet additif. Le polypeptide selon la présente invention comprend de préférence au moins deux mutations qui défavorisent les interactions entre les hélices N/C. Selon un aspect particulièrement préféré ces deux mutations sont combinées avec au moins une mutation, de préférence deux mutations, qui favorisent les interactions entre les hélices N selon une orientation parallèle. Ledit polypeptide ainsi obtenu peut avantageusement comporter en outre une ou plusieurs des autres mutations présentant les effets identifiés à la figure 4, et en particulier au moins une des mutations identifiées dans le tableau 1 aux colonnes 3 à 6. De préférence les positions K40 et D98 ne sont pas mutées simultanément.
Les mutations proposées dans le cadre de la présente invention sont résumées dans le tableau 1.

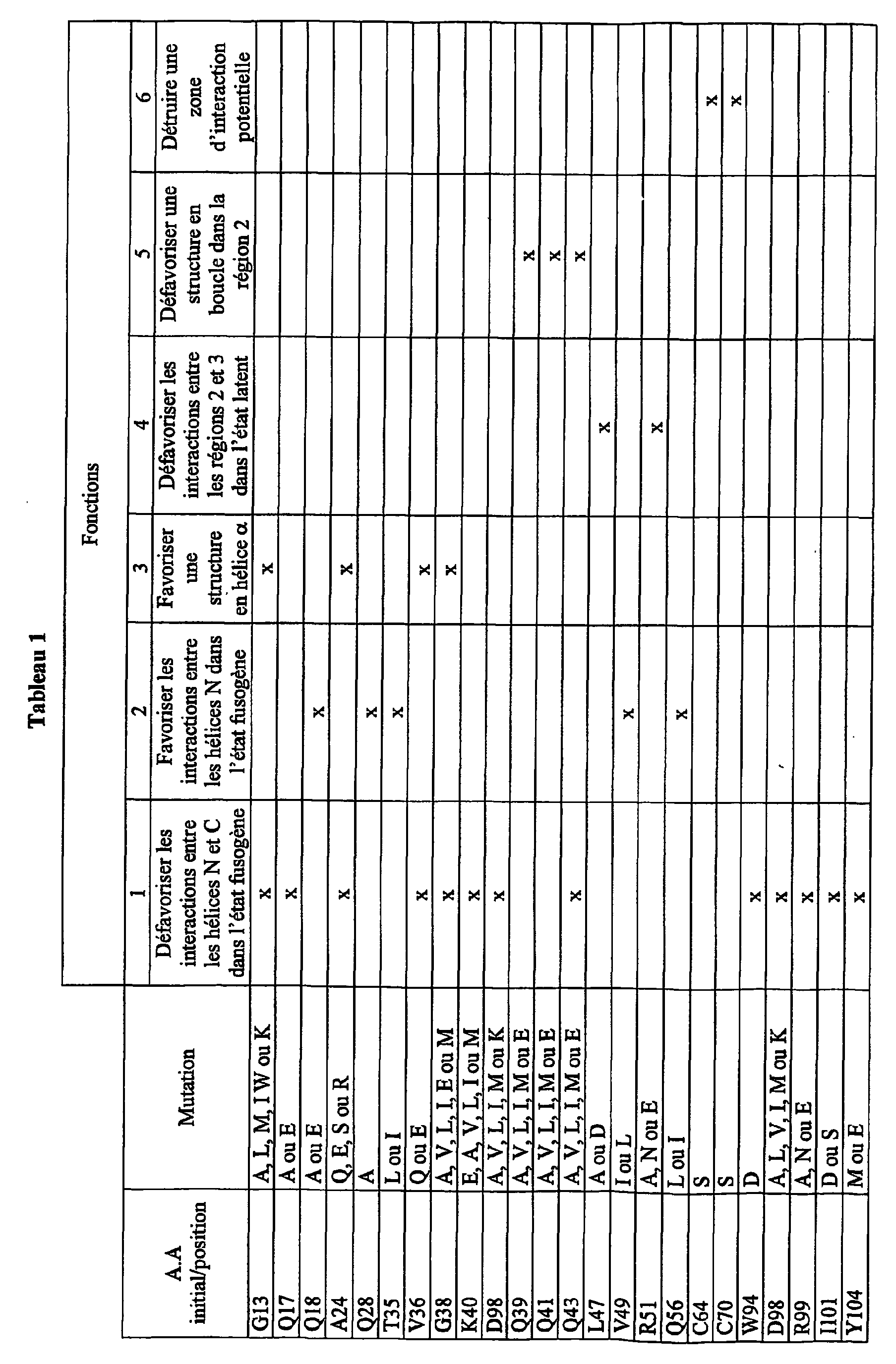

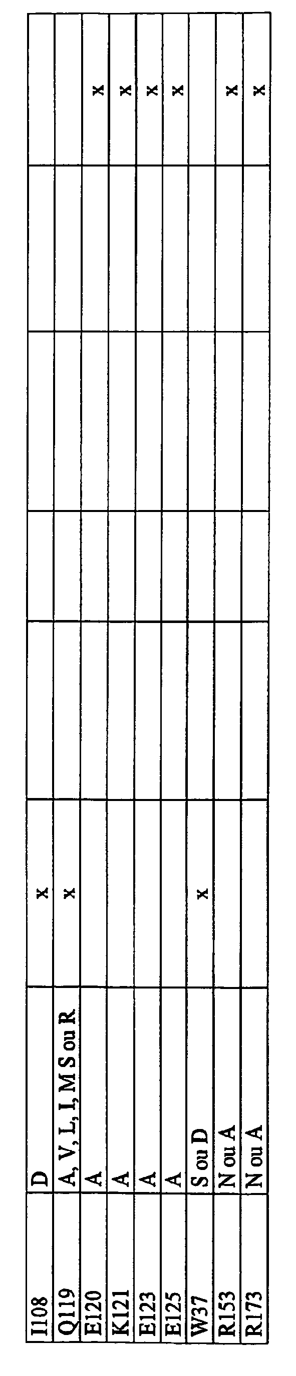

Le polypeptide selon l'invention peut être obtenu par toute technique classique de synthèse chimique ou de génie génétique.
Lorsque le polypeptide est produit par synthèse chimique, le polypeptide selon l'invention peut soit être synthétisé sous la forme d'une séquence unique, soit sous la forme de plusieurs séquences qui sont ensuite liées les unes aux autres. La synthèse chimique peut être réalisée en phase solide ou en solution, ces deux techniques de synthèse étant bien connues de l'homme de l'art. Ces techniques sont décrites notamment par Atherton et Shepard dans « solid phase peptide synthesis (IRL press Oxford, 1989) et par Houbenweyl dans « method der organischen chemie » édité par E.Wunsch vol, 15-I et II thieme, Stuttgart, 1974, ainsi que dans les articles suivants : Dawson PE et al (Synthesis of proteins by native chemical ligation Science 1994 ; 266(5186):776-9); Kochendoerfer GG et al (Chemical protein synthesis. Curr Opin Chem Biol 1999 ; 3(6):665-71); et Dawson PE et al Synthesis of native proteins by chemical ligation, Annu Rev Biochem 2000 ; 69:923-60.
Le polypeptide selon l'invention peut être également produit par les techniques de génie génétique bien connues de l'homme de l'art. Ces techniques sont décrites en détails dans Molecular Cloning : a molecular manual de Maniatis et al, Cold Spring Harbor, 1989). Classiquement, la séquence d'ADN codant pour le polypeptide selon l'invention est insérée dans un vecteur d'expression, mutée par mutagénèse dirigée. Lorsque que plusieurs mutations doivent être introduite, une première réaction de mutagenèse est réalisée, puis le plasmide muté résultant est utilisé comme matrice pour la réalisation de la deuxième réaction de mutagenèse afin d'obtenir le plasmide comprenant la double mutation. Lorsque 2 mutations sont séparées de moins de 5 acides aminés, ces deux mutations sont réalisées simultanément avec un seul oligonucléotide qui porte les deux mutations.
Le vecteur d'expression renfermant la séquence mutée est ensuite utilisé pour transformer une cellule hôte permettant l'expression de la séquence d'intérêt. Le polypeptide produit est ensuite isolé du milieu de culture par des techniques classiques bien connues de l'homme de l'art, telles que précipitation à l'éthanol ou au sulfate d'ammonium, extraction par l'acide, chromatographie d'échange d'anion/cation, chromatographie sur phosphocellulose, chromatographie d'interaction hydrophobe, chromatographie d'affinité, chromatographie sur hydroxyapatite et chromatographie sur lectine. De préférence, la chromatographie liquide à haute performance (HPLC) est utilisée dans la purification.
Selon le système expression utilisé (protéine sécrétée ou non) et selon le procédé de purification, le polypeptide purifié peut se présenter sous différentes formes. Il peut être sous une forme dénaturée ou non dénaturée, monomérique ou multimérique. Lorsqu'il se trouve sous une forme dénaturée il est possible de lui rendre sa conformation ouverte selon l'invention en utilisant le procédé décrit dans l'exemple 1. Pour obtenir des formes multimériques et en particulier des trimères les molécules de polypeptide purifiées doivent être placées dans un milieu qui permet aux molécules d'être parfaitement solubles et essentiellement sans interaction entre elles et de préférence sans structure secondaire. Pour cela on peut utiliser des détergents tels que dodecyl sulfate de sodium, N lauryl sarcosine, Guanidinium chloride, Urée, Thiocyanate de sodium ou des agents chaotropiques. On pourra favoriser les conditions désirées par l'utilisation de solvants organiques ou l'utilisation d'acides. Cette première condition étant satisfaite, on place l'échantillon dans une cassette de dialyse pour éliminer une partie des agents chaotropiques, de façon à favoriser les interactions entre les monomères de polypeptide en conservant aux molécules suffisamment de solubilité. Dans une deuxième étape, la formation des trimères étant favorisée, on dialyse complètement l'échantillon dans un milieu physiologique qui maintient le polypeptide en solution ou en suspension. On obtient alors des trimères du polypeptide selon l'invention dans une conformation ouverte. Une telle technique est décrite en détail dans WO00/08167.

Tout vecteur d'expression classiquement utilisé pour l'expression d'une protéine recombinante peut être utilisé dans le cadre de la présente invention. Ce terme englobe donc aussi bien les vecteurs d'expression dits « vivants » tels que les virus et les bactéries que les vecteurs d'expression de type plasmidiques.

On utilise de préférence des vecteurs dans lesquels la séquence d'ADN du polypeptide selon l'invention est sous la dépendance d'un promoteur fort, inductible ou non inductible. On peut citer à titre d'exemple de promoteur utilisable, le promoteur de l'ARN polymérase T7.

Les vecteurs d'expression incluent de préférence au moins un marqueur de sélection. De tels marqueurs incluent, par exemple, la dihydrofolate réductase ou la résistance à la néomycine pour la culture de cellule d'eucaryote et les gènes de résistance à la kanamycine, tétracycline ou ampicilline pour la culture dans *E. coli* et autres bactéries.

On peut citer à titre de vecteur d'expression utilisable dans le cadre de la présente invention les plasmides pET28 (Novagen) ou pBAD (Invitrogen) par exemple ; les vecteurs viraux tels que : baculovirus, poxvirus en particulier les poxvirus décrits dans les brevets US 5,942,235, US 5,756,103 et US 5,990,091, les virus de la vaccine recombinants, en particulier les virus recombinants décrits dans les brevets EP 83286, US 5,494,807 et US 5,762,938.

La mutagénèse dirigée est réalisée selon les techniques usuelles couramment utilisées par l'homme de l'art, par exemple, en utilisant la polymérase *Pfu* (Quich Change Mutagenesis Kit, Stratégène) ou le kit de mutagénèse de Bio-Rad. Les mutations sont confirmées par séquençage de la façon usuelle. Ce type de méthode est décrit en détail dans le Maniatis et al (Molecular cloning, a laboratory manual cf supra).

Pour favoriser l'expression et la purification du polypeptide, ce dernier peut être exprimé dans une forme modifiée, telle qu'une protéine de fusion, et peut inclure non seulement des signaux de sécrétion, mais aussi des régions fonctionnelles hétérologues supplémentaires. Par exemple, une région d'acides aminés supplémentaires, particulièrement des acides aminés chargés, peut être ajoutée au N-terminal du polypeptide pour améliorer la stabilité et le maintien dans la cellule hôte.

Pour l'expression du polypeptide, toute cellule hôte classiquement utilisée en association avec les vecteurs d'expression décrits ci-dessus peut être utilisée.

On peut citer à titre d'exemple non limitatif les cellules de E. coli, BL21 (λDE3), HB101, Topp 10, CAG 1139, Bacillus, les cellules eucaryotes telles que CHO ou Vero.

De préférence on utilisera dans le cadre de la présente invention le système vecteur d'expression /cellule suivant : pET(Cer) /BL21LamdaDE3, ou BL211amdaDE3(RIL).

En fonction de l'hôte employé dans la procédure de production par voie recombinante, les polypeptides de la présente invention peuvent être glycosylés ou non-glycosylés. En plus, les polypeptides de l'invention peuvent aussi inclure un résidu de méthionine supplémentaire en N-terminal.

La présente invention a également pour objet les conjugués comprenant un polypeptide selon l'invention et une protéine porteur ou un peptide porteur.

La protéine (ou peptide) porteur renforce l'immunogénicité du polypeptide selon l'invention notamment en augmentant la production d'anticorps spécifiques. Ladite protéine (ou peptide) porteur comprend de préférence un ou plusieurs épitope(s) T helper. Par « épitope T helper », on entend un enchaînement d'acides aminés qui, dans le contexte d'une ou plusieurs molécules du MHC classe II, active les lymphocytes T helper. Selon un mode de réalisation avantageux, la protéine (ou peptide) porteur utilisée améliore la solubilité dans l'eau du polypeptide selon l'invention.

Comme protéine porteur on peut utiliser par exemple les protéines de surface de phages comme la protéine pIII ou pVIII du phage M13, les protéines de surface bactériennes comme les protéines LamB, OmpC, ompA, ompF et PhoE d'E. coli, la protéine CotC ou CotD de B. Subtilis, les porines bactériennes comme la porine P1 de Neisseria gonorrheae, la porine P1 ou P2 d'H. influenzae B, la porine de classe I de N. meningitidis B, la porine P40 de K. pneumoniae, des lipoprotéines telles que OspA de B. bugdorfi, PspA de S. pneumoniae, TBP2 de N. meningitidis B, TraT d'E. coli ainsi que l'adhésine A de S. pneumoniae ; les « heat shock » protéines telles que Hsp65 ou Hsp71 de M. tuberculosis ou bovis ou Hin 47 d'H. infuenzae type B. Sont également particulièrement appropriées dans le cadre de la présente invention, les toxines bactériennes détoxifiées comme l'anatoxine tétanique ou diphtérique, la sous unité B de la toxine cholérique, la sous unité B de la toxine cholérique, la sous unité B de l'endotoxine A de P. aeruginosa ou l'exotoxine A de S. aureus.

Comme peptide porteur on peut utiliser par exemple dans le cadre de la présente invention les peptides p24E, p24N, p24H et p24M décrits dans WO94/29339 ainsi que les peptides PADRE tels que décrits par Del guercio et al (Vaccine (1997) ; vol15/4, p441-448).

La protéine (ou peptide) porteur est liée à l'extrémité N ou C terminale du polypeptide selon l'invention par tout procédé de conjugaison bien connu de l'homme de l'art. De plus, la séquence codant pour la protéine (ou peptide) porteur peut avantageusement être fusionnée à la séquence codant pour le polypeptide selon l'invention et la séquence résultante peut être exprimée sous la forme d'une protéine de fusion par tout procédé classique. Toutes les techniques de génie génétique utiles pour ce faire sont décrites dans le Maniatis et al. Lesdits conjugués peuvent être isolés par tout procédé classique de purification bien connus de l'homme de l'art.

La présente invention a également pour objet les séquences d'ADN codant pour les polypeptides et les conjugués selon l'invention ainsi que les vecteurs d'expression comprenant lesdites séquences et les cellules hôtes transformées par lesdits vecteurs.

Plutôt que d'extraire et de purifier le polypeptide ou le conjugué exprimé par le vecteur d'expression il est souvent plus facile et parfois plus avantageux d'utiliser le vecteur d'expression lui-même dans le vaccin selon l'invention. La présente invention a donc également pour objet tout vecteur d'expression tel que défini ci-dessus.

Toute cellule hôte telle que définie ci-dessus transformée par un tel vecteur d'expression est incluse dans le cadre de la présente invention.

La présente invention a également pour objet les anticorps dirigés contre les polypeptides et conjugués tels que décrits ci-dessus. La préparation de tels anticorps est mise en oeuvre par les techniques classiques d'obtention d'anticorps polyclonaux et monoclonaux bien connues de l'homme de l'art.

Ces anticorps sont particulièrement appropriés pour être utilisés dans un schéma d'immunisation passive.

La présente invention a également pour objet des vaccins utiles à des fins thérapeutiques et prophylactiques. Les vaccins selon la présente invention comprennent au moins un polypeptide, au moins un conjugué ou au moins un vecteur d'expression tel que défini ci-dessus, un support ou diluant pharmaceutiquement acceptable et éventuellement un adjuvant.

La quantité de polypeptide, de conjugué ou de vecteur dans le vaccin selon la présente invention dépend de nombreux paramètres comme le comprendra l'homme de l'art, tels que la nature de la protéine porteur, le vecteur utilisé ou, la voie d'administration. Une quantité appropriée est une quantité telle qu'une réponse immunitaire humorale capable de neutraliser des isolats primaires du VIH est induite après administration de cette dernière. La quantité de polypeptide à administrer est de l'ordre de 10 à 100 micro grammes. La quantité de conjugué à administrer sera déduite des quantités indiquées ci-dessus en tenant compte du PM de la protéine porteur. La quantité de vecteur d'expression à administrer est de l'ordre de 10 à 5000 micro grammes dans le cas d'un vecteur non viral et de l'ordre de 10^{E}4 à 10^{E}8 TCID50 dans le cas d'un vecteur viral.

Les vaccins selon la présente invention peuvent également contenir un adjuvant. Tout adjuvant ou mélange d'adjuvants pharmaceutiquement acceptable peut être utilisé à cette fin. On peut citer à titre d'exemple les sels d'aluminium tels que l'hydroxyde d'aluminium ou le phosphate d'aluminium. Des agents auxiliaires classiques tels que agents mouillants, charges, émulsifiants, tampons etc. peuvent également être additionnés au vaccin selon l'invention.

Les vaccins selon la présente invention peuvent être préparés par tout procédé classique connu de l'homme de d'art. Classiquement les antigènes selon l'invention sont mélangés avec un support ou diluant pharmaceutiquement acceptable, tel que eau ou solution saline tamponnée au phosphate. Le support ou diluant va être sélectionné en fonction de la forme galénique choisie, du mode et de la voie d'administration ainsi que de la pratique pharmaceutique. Les supports ou diluants appropriés ainsi que les exigences en matière de formulation pharmaceutique sont décrits en détails dans Remington's Pharmaceutical Sciences, représentant un ouvrage de référence dans ce domaine.

Les vaccins mentionnés ci-dessus peuvent être administrés par toute voie classique, habituellement utilisée dans le domaine des vaccins, telle que la voie parentérale (intraveineuse, intramusculaire, sous-cutanée, etc..). On utilisera de préférence dans le cadre de la présente invention une administration intramusculaire. Une telle administration peut être réalisée avantageusement au niveau des muscles de la cuisse ou du bras. Une administration par voie muqueuse nasale, orale, vaginale ou rectale peut être également recommandée dans le cadre de la présente invention. L'administration peut être réalisée par l'administration d'une dose unique ou de doses répétées, par exemple à J0, à 1 mois, à 3 mois, à 6 mois et à 12 mois. On utilisera de préférence les injections à J0 , à 1 mois et à 3 mois avec un rappel dont la périodicité pourra être aisément déterminée par le médecin traitant.

Le vaccin selon la présente invention peut avantageusement être administré selon un schéma posologique comprenant la co-administration d'un vecteur d'expression selon l'invention et d'un polypeptide selon l'invention ou selon un schéma de « prime-boost » dans lequel le vecteur selon l'invention est administré en premier et le polypeptide en tant qu'injection de rappel. Dans ces deux schéma posologique, le vecteur d'expression selon l'invention peut être remplacé par tout vecteur d'expression exprimant un ou plusieurs antigènes ou épitopes du VIH différents du polypeptide selon l'invention, et en particulier par un vecteur ALVAC ou NYVAC.

La présente invention entend également couvrir un polypeptide, un conjugué ou un vecteur tel que défini ci-dessus et le vaccin contenant ces composés pour leur utilisation pour induire des anticorps neutralisant des isolats primaires du VIH.

La demanderesse a mis en évidence de façon surprenante que le polypeptide selon l'invention étaient capables après administration d'induire des anticorps susceptibles de neutraliser des isolats primaires du VIH. Ces antigènes représentent donc des candidats de valeur pour l'élaboration d'un vaccin utilisable pour la protection et/ou le traitement d'un grand nombre, voire la totalité des sujets à risques ou infectés par le VIH.

La présente invention concerne donc également une méthode d'induction d'une réponse immunitaire chez un sujet hôte incluant l'homme comprenant l'administration d'un vaccin selon l'invention. On entend par «une réponse immunitaire » une réponse comprenant la production d'anticorps dirigés spécifiquement contre le polypeptide selon l'invention. La production d'anticorps spécifiques peut être aisément déterminée par des techniques classiques bien connues de l'homme de l'art telles que ELISA, RIA, western blot.

L'invention a également pour objet une méthode de diagnostic comprenant la mise en contact d'un polypeptide selon l'invention avec un échantillon biologique et la détection des complexes anticorps/polypeptide formés.

Le polypeptide selon la présente invention peut en effet être utilisé dans un test ELISA pour détecter les anticorps anti-gp41 qui sont présents dans le sérum des individus. Le polypeptide selon la présente invention est donc utile comme outil de diagnostic car la présence d'anticorps anti-gp41 est un marqueur fiable d'une infection par le HIV.

Dans ce cas, le polypeptide selon l'invention est déposé sur une plaque ELISA, puis mis en contact avec des dilutions en série du sérum du patient à tester et enfin mis en contact avec un anticorps anti-humain lié à une enzyme. Le complexe anticorps anti-humain/anticorps anti-gp41/polypeptide ainsi formé est ensuite détecté par colorimétrie.

La présente invention va être décrite de façon plus détaillée dans les exemples qui suivent par référence aux figures annexées dans lesquelles :
La figure 1 est une représentation schématique du phénomène de changement de conformation de la gp41 qui précède la fusion des membranes cellulaire et virale.
La figure 2 donne la séquence complète de la gp41 LAI dans laquelle (_____) représente le peptide de fusion et (------) représente le domaine transmembranaire.
La figure 3 donne la séquence du polypeptide dérivant de la protéine gp41 LAI qui est utilisé comme produit de départ dans les exemples fournis.
Les figures 4 et 5 donnent une représentation schématique des régions 1 à 4, la figure 4 résume les fonctions des mutations envisagées. Les chiffres donnés entre parenthèses se référent aux régions identifiées, « favoriser les interactions (3-12) » signifiant que les interactions entre la région 3 et les régions 1 et 2 sont favorisées.

Les exemples décrits ci-dessous sont donnés à titre purement illustratif de l'invention et ne peuvent en aucun cas être considérés comme limitant la portée de cette dernière.

### Exemple 1 : Préparation de différents polypeptides selon l'invention

### 1- clonage de la séquence de la figure 3 dans un vecteur d'expression

La séquence d'ADN codant pour le polypeptide identifié à la figure 3 a été clonée dans un système d'expression inductible.

Le vecteur utilisé est le Pet -cer qui est construit à partir du vecteur pET28 de Novagen. Le vecteur commercial pET28c a été amplifié par PCR à l'aide de 2 amorces situées de part et d'autre de la région correspondant à l'origine F1, de sorte que le produit amplifié corresponde à la quasi totalité du vecteur d'origine moins la région comprenant l'origine F1. Les sites de restriction uniques AscI et PacI sont apportés respectivement par les 2 amorces ayant servi à l'amplification. Parallèlement le fragment cer est amplifié à l'aide de 2 amorces qui permettent d'obtenir ce fragment encadré par les sites AscI et PacI.

Vecteur et fragment Cer sont digérés par les enzymes AscI et PacI puis ligués entre eux Ce vecteur comprend en particulier une cassette d'expression sous le contrôle du promoteur T7, un polylinker en aval du promoteur T7 pour le clonage du gène d'intérêt, le fragment CER situé en aval du polylinker , permettant de diminuer la multimérisation des plasmides, un terminateur de transcription T7 term et le gène de résistance à la kanamycine. La régulation positive du promoteur est obtenue en présence de la T7 RNA polymérase.

### 2- mutagénèse dirigée

La mutagénèse dirigée pour l'obtention des polypeptides mutés selon l'invention est mise en oeuvre par utilisation du KIT QuickChange site-directed mutagenesis de Stratagène.

Pour chaque mutation 2 oligonucléotides de mutagénése qui encadre l'acide aminé à muter sont définis. Par exemple pour la mutation R51A, les oligonucléotides suivants sont utilisés :

R
Séquence de référence ctg gct gtg gaa aga tac cta aag gat
A

Oligonucléotide 5'	ctg gct gtg gaa gca tac cta aag gat

Oligonucléotide 3'	atc ctt tag gta tgc ttc cac agc cag

Les 2 oligonucléotides vont s'hybrider à la même séquence sur les brins complémentaires du plasmide contenant la séquence à muter. La mutation se situe au centre des oligonucléotides et elle est bordée par 12 nucléotides de chaque côté.

La réaction de mutagénèse est réalisée sur le plasmide de l'exemple 1 dans les conditions suivantes : on soumet un mélange contenant : Tampon de réaction 10X 5µl ; plasmide à muter 100ng/µl 1µl ; Oligo 5' (125ng/µl ), 1µl ; Oligo 3' (125ng/µl ), 1µl, Mix dNTPs 10mM, 1µl, H20 UF, 40µl et polymérase thermostable de Pyrococcus furiosus 2,5U/µl, 1µl à une PCR selon les cycles définis ci-dessous : 95°C, 30" ; 95°C, 30" ; 55°C, 1' ; 68°C, 2'/kpb de plasmide ; 12 cycles ; température de fin de réaction : 20°C.

En utilisation le protocole ci-desssus, les différents mutants identifiés dans le tableau 1 ont été préparés.

### 3- expression

L'expression des plasmides issus de l'étape 2 ci-dessus est réalisée chez E.coli. Pour ce faire une souche modifiée d' E.coli est utilisée :BL21 RILλDE3.

Cette souche est enrichie en tRNA rares (ARG, ILE, LEU), elle contient le gène codant pour la T7 RNA polymérase lequel est sous le contrôle du promoteur *lac* UV5 inductible par addition d'IPTG à une concentration de 1mM.

Dans un premier temps la souche est transformée par le plasmide muté selon le protocole comprenant les étapes suivantes : repiquage de 3 colonies dans 10 ml de LB +ANTIBIOTIQUE ; Incubation une nuit à 37°C ; réensemencer la préculture au 1:100 dans 15ml de LB+ANTIBIOTIQUE au 1:100 ; laisser pousser jusqu'à DO600 de 0.5 ; Prélever 1 ml pour vérifier la DO600 ; prélever 7 ml pour l'échantillon non induit ; induire les 7 autres ml avec 1mM d'IPTGet Induction 3H à 37°C.

Le même protocole a été mis en oeuvre sur plusieurs litres de culture pour produire une grande quantité de bactéries pour purifier le polypeptide muté.

### 4- purification

Le culot cellulaire formé des bactéries récoltées dans un litre de milieu de culture est décongelé et repris dans 2x 100ml de tampon Tris 30mM à pH8 en présence d'un inhibiteur de protéase (Péfabloc, Interchim) à la concentration de 100µM. Du lysozyme est ajouté à la concentration de 100µg/ml et le mélange est incubé 30 minutes à température ambiante. Les cellules sont ensuite cassées par sonication

(4 cycles de deux minutes) avec une puissance approximative de 150Watts. La gp41 se trouve sous forme de corps d'inclusion. Ceux-ci sont lavés dans un tampon PBS-tween20 à 0,05% à 4°C et centrifugés pendant 15minutes à 10000g. Après élimination du surnageant, la solubilisation du culot de centrifugation composé essentiellement des corps d'inclusion est effectuée en une heure à température ambiante en agitation douce en présence de 50ml de tampon CAPS à pH 10,4 contenant 3% de N-lauryl sarcosine.

La fraction solubilisée est ensuite dialysée à 4°C contre un tampon Tris 30mM à pH 8 contenant de l'urée 8M (5 bains) et filtrée sur un filtre de porosité 0,45µm puis chargée sur un colonne Hi-Trap 1ml (Pharmacia). Ces supports de chromatographie d'affinité chelatent des atomes de Nickel sur lesquels se fixent les résidus histidines de l'extrémité C terminale de la protéine.

Après lavage, l'élution de la protéine est obtenue dans le tampon Tris 30mM à pH8 contenant de l'urée 8M et 500mM d'imidazole. Les fractions éluées sont dialysées dans un tampon Tris 30mM à pH8 et 8M urée sans imidazole et avec des quantités décroissantes d'urée, en allant jusqu'à 2M. Cette technique permet de purifier toutes les molécules de gp41 mutantes ou natives en présence ou en absence du peptide de fusion.

### Exemple 2 : Immunogénicité et induction d'anticorps neutralisants

L'immunogène testé dans cet exemple correspond à un polypeptide de séquence SEQ ID N°2 dans laquelle la mutation I101D a été introduite par mutagénèse dirigée et qui comprend en C-terminal une séquence d'histidines pour faciliter sa purification. La préparation de l'immunogène a été mise en oeuvre selon les procédés décrits dans les exemples précédents.

Des groupes de 5 cobayes ont été immunisés 3 fois par voie intramusculaire (dans la cuisse, au niveau du muscle *biceps femoris*) à 3 semaines d'intervalle (jours 1, 22 et 43) avec 20 µg par dose de gp41 native ou de polypeptide I101D en présence de 6 mg de phosphate d'aluminium. L'immunogène a été administré sous un volume de 0,5 ml, soit 0,25 ml par cuisse. Les sérums ont été prélevés à J1, J43 et J57.

Les sérums immuns (J43) individuels et les mélanges de sérums préimmuns (J1) de chaque groupe ont été testés en ELISA pour leurs titres anticorps IgG induits contre la gp41 native et contre la gp 160 MN/LAI-2.

Les sérums immuns (J57) individuels et les mélanges de sérums préimmuns (J1) de deux groupes ont été testés pour leur activité seroneutralisante vis à vis de 1' isolat VIH-1 primaire Bx08.

Les résultats obtenus montrent que le polypeptide selon la présente invention est aussi immunogène que la gp41 native.

De plus, le test de neutralisation de C. Moog et al a montré que le polypeptide selon l'invention induisait des anticorps neutralisants. (% de réduction > à un facteur 10).

### LISTE DE SEQUENCES

<110> AVENTIS PASTEUR
<120> POLYPEPTIDE INDUISANT DES ANTICORPS NEUTRALISANT LE VIH
<130> PM0101
<140>
   <141>
<160> 2
<170> PatentIn Ver. 2.1
<210> 1
   <211> 344
   <212>
   <213> Human immunodeficiency virus type 1
<220>
   <221> PEPTIDE
   <222> (1)..(344)
   <223> protéine gp41 LAI
<400> 1 
avgigalflg flgaagstmg aasmtltvqa rqllsgivqq qnnllraiea qqhllqltvw 60
gikqlqaril averylkdqq llgiwgcsgk licttavpwn aswsnksleq iwnhttwmew 120
dreinnytsl ihslieesqn qqekneqell eldkwaslwn wfnitnwlwy iklfimivgg 180
lvglrivfav lsivnrvrqg ysplsfqthl ptprgpdrpe gieeeggerd rdrsirlvng 240
slaliwddlr slclfsyhrl rdlllivtri vellgrrcwe alkywwnllq vwselknsav 300
sllnataiav aegtdrviev vqgacrairh iprrirqgle rill 344
<210> 2
   <211> 177
   <212>
   <213> Human immunodeficiency virus type 1
<220>
   <221> PEPTIDE
   <222> (1) .. (177)
   <223> polypeptide dérivé de gp 41 LAI
<400> 2

mtltvqarql lsgivqqqnn llraieaqqh llqltvwgik qlqarilave rylkdqqllg 60
iwgcsgklic ttavpwnasw snksleqiwn nmtwmewdre innytslihs lieesqnqqe 120
kneqelleld kwaslwnwfn itnwlwyikn rvrqgyspls fqthlptprg pdrpegi 177

## Revendications

1. Polypeptide de séquence SEQ ID N°2 comprenant au moins une mutation sélectionnée dans le groupe consistant en : I101D ou S.

2. Polypeptide selon la revendication 1 comprenant au moins une autre mutation sélectionnée dans le groupe comprenant : G13A, L, M, I, W ou K ; Q 17A ou E ; Q 18A ou E; A24Q, E, S ou R ; Q28A ; T35I ou L ; V36Q ou E ; W37S ou D ; G38A, V, L, I, M ou E ; Q39A, V, L, I, M ou E ; K40E, A, V, L, I ou M ; Q41A, V, L, I, M ou E ; Q43A, V, L, I, M ou E ; L47A ou D ; V49I ou L ; R51A, N ou E ; Q56I ; C64S ; C70S ; ou L ; W94D ; D98A, V, L, I, M ou K ; R99A, N ou E ; Y104M ou E ; I108D ; Q119A, V, L, I, M, S, N ou R ; E120A ; K121A ; E123A ; E125A ; R153N ou A et R173N ou A.

3. Polypeptide selon l'une quelconque des revendications 1 à 2, comprenant les mutations suivantes : T35I + Q28I +I101D ; T35I + Q28I + 1101D + Q119N ; I101D + I108D + Q131N+W37A; 1101D + I108D + Q142N + L126D ; W37A + I101D + I108D + Q119N; ou I101D + I108D + Q119N + L126D.

4. Conjugué comprenant un polypeptide selon l'une des revendications 1 à 3, conjugué à une protéine ou un peptide porteur.

5. Séquence d'ADN codant pour un polypeptide selon l'une quelconque des revendications 1 à 3 ou pour un conjugué selon la revendication 4.

6. Vecteur d'expression comprenant la séquence d'ADN selon la revendication 5.

7. Cellule-hôte contenant le vecteur selon la revendication 6.

8. Procédé de préparation d'un polypeptide selon l'une quelconque des revendications 1 à 3 ou un conjugé selon la revendication 4 comprenant l'expression dudit polypeptide à partir d'une cellule-hôte telle que définie dans la revendication 7.

9. Vaccin contre le VIH, comprenant au moins un polypeptide selon l'une des revendications 1 à 3, ou au moins un conjugué selon la revendication 4 ou au moins un vecteur d'expression selon la revendication 6, un support pharmaceutiquement acceptable et éventuellement un adjuvant.

10. Utilisation d'un polypeptide selon l'une quelconque des revendications 1 à 3 pour la préparation d'un médicament pour l'induction d'anticorps neutralisant des isolats primaires du VIH.

## Patentansprüche

1. Polypeptid mit der Sequenz SEQ ID NO: 2, umfassend wenigstens eine Mutation, die ausgewählt ist aus der Gruppe, die besteht aus: I101D oder S.

2. Polypeptid gemäß Anspruch 1, umfassend wenigstens eine weitere Mutation, die ausgewählt ist aus der Gruppe, die umfasst: G13A, L, M, I, W oder K; Q17A oder E; Q18A oder E; A24Q, E, S oder R; Q28A; T351 oder L; V36Q oder E; W37S oder D; G38A, V, L, I, M oder E; Q39A, V, L, I, M oder E; K40E, A, V, L, I oder M; Q41A, V, L, I, M oder E; Q43A, V, L, I, M oder E; L47A oder D; V491 oder L; R51A, N oder E; Q561; C64S; C70S oder L; W94D; D98A, V, L, I, M oder K; R99A, N oder E; Y104M oder E; I108D; Q119A, V, L, I, M, S, N oder R; E120A; K121A; E123A; E125A; R153N oder A und R173N oder A.

3. Polypeptid gemäß einem der Ansprüche 1 bis 2, umfassend die folgenden Mutationen: T35I + Q28I + I101D; T35I + Q28I + I101D + Q119N; I101D + I108D + Q131N + W37A; I101D + I108D + Q142N + L126D; W37A + I101D + I108D + Q119N; oder I101D + 1108D + Q119N + L126D.

4. Konjugat, umfassend ein Polypeptid gemäß einem der Ansprüche 1 bis 3, konjugiert an ein Trägerprotein oder -peptid.

5. DNA-Sequenz, die für ein Polypeptid gemäß einem der Ansprüche 1 bis 3 oder für ein Konjugat gemäß Anspruch 4 kodiert.

6. Expressionsvektor, umfassend die DNA-Sequenz gemäß Anspruch 5.

7. Wirtszelle, die den Vektor gemäß Anspruch 6 enthält.

8. Verfahren zur Herstellung eines Polypeptids gemäß einem der Ansprüche 1 bis 3 oder eines Konjugats gemäß Anspruch 4, umfassend die Expression besagten Polypeptids aus einer wie in Anspruch 7 definierten Wirtszelle.

9. Impfstoff gegen HIV, umfassend wenigstens ein Polypeptid gemäß einem der Ansprüche 1 bis 3 oder wenigstens ein Konjugat gemäß Anspruch 4 oder wenigstens einen Expressionsvektor gemäß Anspruch 6, einen pharmazeutisch annehmbaren Träger und gegebenenfalls ein Adjuvans.

10. Verwendung eines Polypeptids gemäß einem der Ansprüche 1 bis 3 zur Herstellung eines Arzneimittels für die Induktion von Antikörpern, die primäre HIV-Isolate neutralisieren.

## Claims

1. Polypeptide of sequence SEQ ID No. 2 comprising at least one mutation selected from the group consisting of: I101D or S.

2. Polypeptide according to Claim 1, comprising at least one other mutation selected from the group comprising: G13A, L, M, I, W or K; Q17A or E; Q18A or E; A24Q, E, S or R; Q28A; T35I or L; V36Q or E; W37S or D; G38A, V, L, I, M or E; Q39A, V, L, I, M or E; K40E, A, V, L, I or M; Q41A, V, L, I, M or E; Q43A, V, L, I, M or E; L47A or D; V49I or L; R51A, N or E; Q56I; C64S; C70S; or L; W94D; D98A, V, L, I, M or K; R99A, N or E; Y104M or E; I108D; Q119A, V, L, I, M, S, N or R; E120A; K121A; E123A; E125A; R153N or A and R173N or A.

3. Polypeptide according to either one of Claims 1 and 2, comprising the following mutations: T35I + Q28I + I101D; T35I + Q28I + I101D + Q119N; I101D + I108D + Q131N + W37A; I101D + I108D + Q142N + L126D; W37A + I101D + I108D + Q119N; or I101D + I108D + Q119N + L126D.

4. Conjugate comprising a polypeptide according to one of Claims 1 to 3, conjugated to a carrier protein or peptide.

5. DNA sequence encoding a polypeptide according to any one of Claims 1 to 3 or encoding a conjugate according to Claim 4.

6. Expression vector comprising the DNA sequence according to Claim 5.

7. Host cell containing the vector according to Claim 6.

8. Process for preparing a polypeptide according to any one of Claims 1 to 3 or a conjugate according to Claim 4, comprising the expression of said polypeptide using a host cell as defined in Claim 7.

9. Vaccine against HIV, comprising at least one polypeptide according to one of Claims 1 to 3, at least one conjugate according to Claim 4 or at least one expression vector according to Claim 6, a pharmaceutically acceptable support and, optionally, an adjuvant.

10. Use of a polypeptide according to any one of Claims 1 to 3, for preparing a medicinal product for inducing antibodies which neutralize primary isolates of HIV.
